# EUROPEAN PATENT APPLICATION

(11) **EP 4 362 031 A1**
(43) Date of publication of application: **01.05.2024**
(21) Application number: 22828446.9
(22) Date of filing: 22.06.2022
(51) Int. Cl.: G16C 20/30, G16C 20/70

(54) **PHYSICAL PROPERTY PREDICTION DEVICE, PHYSICAL PROPERTY PREDICTION METHOD, AND PROGRAM**

(30) Priority: 23.06.2021 JP 2021104173
(71) Applicant: Resonac Corporation, Tokyo (JP)
(72) Inventor: AONUMA, Naoto, Tokyo 105-8518 (JP); TAKEMOTO, Shimpei, Tokyo 105-8518 (JP); MINAMI, Takuya, Tokyo 105-8518 (JP); KAKUDA, Kohsuke, Tokyo 105-8518 (JP); OKUNO, Yoshishige, Tokyo 105-8518 (JP); TAKASHI, Hiroko, Tokyo 105-8518 (JP)
(74) Representative: Strehl Schübel-Hopf & Partner
(86) International application number: PCT/JP2022/024845
(87) International publication number: WO 2022/270530

(57) **Abstract**

A physical property prediction device for predicting a physical property of a compound including: a generation unit configured to generate a first stage trained model, by using, as training data, first stage synthesis information and synthesis result information; a generation unit configured to generate second stage through last stage trained models, by using, as training data, n-th stage (n≥2) synthesis information, n-th stage synthesis result information, and n-1-th stage synthesis result information; a reception unit configured to receive a synthesis information setting for the compound for which the physical property is predicted; a prediction unit configured to predict a physical property value of a product synthesized by the first stage chemical reaction of the compound; a prediction unit configured to repeat, from a second stage chemical reaction to a last stage chemical reaction of the compound, a process of predicting a physical property value of a product synthesized by the n-th stage chemical reaction of the compound; and an output unit configured to output a predicted physical property value.

## Description

### TECHNICAL FIELD

The present disclosure relates to a physical property prediction device, a physical property prediction method, and a program.

### BACKGROUND ART

In recent years, physical properties of organic compounds such as polymers have been predicted by machine learning using computers. For example, in Patent Document 1, a technique is proposed in which a regression model for predicting physical property values is constructed using experimental values of physical properties of polymers and number densities of substructures of the polymers, and physical property values corresponding to an input polymer structure are predicted using the regression model (see, for example, Patent Document 1).

### PRIOR ART DOCUMENT

### PATENT DOCUMENT

Patent Document 1: Japanese Patent No. 6633820

### DISCLOSURE OF THE INVENTION

### PROBLEM TO BE SOLVED BY THE INVENTION

For example, some compounds, such as polymers, are synthesized by multi-stage chemical reactions such as polymerization reactions and addition reactions. There is a need for technology that can accurately predict the physical properties of compounds synthesized through such multi-stage chemical reactions.

One object of the present disclosure is to provide a physical property prediction device, a physical property prediction method, and a program capable of accurately predicting the physical properties of compounds synthesized by multi-stage chemical reactions.

### MEANS OF SOLVING THE PROBLEM

The present disclosure includes the following configurations.
[1] A physical property prediction device for predicting a physical property of a compound synthesized by multi-stage chemical reactions, the physical property prediction device including:
   a first model generation unit configured to generate a first stage trained model for predicting a physical property value of a product synthesized by a first stage chemical reaction, by using, as training data, synthesis information about the first stage chemical reaction and synthesis result information about a product synthesized by the first stage chemical reaction;
   a second model generation unit configured to generate second stage through last stage trained models for predicting a physical property value of a product synthesized by an n-th stage (n≥2) chemical reaction, by using, as training data, synthesis information about the n-th stage chemical reaction, synthesis result information about a product synthesized by the n-th stage chemical reaction, and synthesis result information about a product synthesized by an n-1-th stage chemical reaction;
   a synthesis information setting reception unit configured to receive a synthesis information setting for each of the multi-stage chemical reactions of the compound for which the physical property is predicted;
   a first prediction unit configured to input synthesis information about the first stage chemical reaction of the compound into the first stage trained model, thereby to predict a physical property value of a product synthesized by the first stage chemical reaction of the compound;
   a second prediction unit configured to repeat, from a second stage chemical reaction to a last stage chemical reaction of the compound, a process of inputting synthesis information about the n-th stage chemical reaction of the compound and synthesis result information about a product synthesized by the n-1-th stage chemical reaction of the compound, into the n-th stage trained model, thereby predicting a physical property value of a product synthesized by the n-th stage chemical reaction of the compound; and
   an output unit configured to output a predicted physical property value of a product synthesized by a last stage chemical reaction of the compound, predicted by the second prediction unit.
[2] The physical property prediction device according to [1], wherein
   the second model generation unit is configured to generate second stage through last stage trained models for predicting a physical property value of a product synthesized by each of the second stage through last stage chemical reactions, by using, as training data, synthesis information about the n-th stage chemical reaction, synthesis result information about a product synthesized by the n-th stage chemical reaction, synthesis information about a chemical reaction of a stage prior to the n-th stage, and synthesis result information about a product synthesized by the chemical reaction of the stage prior to the n-th stage, and
   the second prediction unit is configured to repeat, to the last stage chemical reaction of the compound, a process of inputting synthesis information about chemical reactions from the first stage chemical reaction to the n-th stage chemical reaction of the compound and synthesis result information about each of products synthesized by chemical reactions from the first stage chemical reaction to the n-1-th stage chemical reaction of the compound, into the n-th stage trained model, thereby predicting the physical property value of the product synthesized by the n-th stage chemical reaction of the compound.
[3] The physical property prediction device according to [1] or [2], wherein
   the synthesis result information about the product includes a feature calculated from the predicted physical property value of the product synthesized by the chemical reaction and from the synthesis information about the product.
[4] The physical property prediction device according to [3], wherein
   the compound synthesized by the multi-stage chemical reactions is a polymer that is polymerized from multiple types of monomers, and the feature includes at least one of a model structure, a number of substructures, a number of atoms in the model structure, and a number density of the substructures of the polymer.
[5] The physical property prediction device according to [3] or [4], wherein
   the physical property value includes, as information, at least one of viscosity, glass transition point, color difference, molecular weight, acid value, developability, adhesiveness, transparency, sensitivity, solvent resistance, dispersibility, and heat resistance of the product.
[6] A physical property prediction method executed by a computer for predicting a physical property of a compound synthesized by multi-stage chemical reactions, the physical property prediction method including:
   a step of generating a first stage trained model for predicting a physical property value of a product synthesized by a first stage chemical reaction, by using, as training data, synthesis information about the first stage chemical reaction and synthesis result information about a product synthesized by the first stage chemical reaction;
   a step of generating second stage through last stage trained models for predicting a physical property value of a product synthesized by an n-th stage (n≥2) chemical reaction, by using, as training data, synthesis information about the n-th stage chemical reaction, synthesis result information about a product synthesized by the n-th stage chemical reaction, and synthesis result information about a product synthesized by an n-1-th stage chemical reaction;
   a step of receiving a synthesis information setting for each of the multi-stage chemical reactions of the compound for which the physical property is predicted;
   a step of inputting synthesis information about the first stage chemical reaction of the compound into the first stage trained model, thereby predicting a physical property value of a product synthesized by the first stage chemical reaction of the compound;
   a step of repeating, from a second stage chemical reaction to a last stage chemical reaction of the compound, a process of inputting synthesis information about the n-th stage chemical reaction of the compound and synthesis result information about a product synthesized by the n-1-th stage chemical reaction of the compound, into the n-th stage trained model, thereby predicting a physical property value of a product synthesized by the n-th stage chemical reaction of the compound; and
   a step of outputting a predicted physical property value of a product synthesized by a last stage chemical reaction of the compound.
[7] A program for causing a computer for predicting a physical property of a compound synthesized by multi-stage chemical reactions, to execute:
   a step of generating a first stage trained model for predicting a physical property value of a product synthesized by a first stage chemical reaction, by using, as training data, synthesis information about the first stage chemical reaction and synthesis result information about a product synthesized by the first stage chemical reaction;
   a step of generating second stage through last stage trained models for predicting a physical property value of a product synthesized by an n-th stage (n≥2) chemical reaction, by using, as training data, synthesis information about the n-th stage chemical reaction, synthesis result information about a product synthesized by the n-th stage chemical reaction, and synthesis result information about a product synthesized by an n-1-th stage chemical reaction;
   a step of receiving a synthesis information setting for each of the multi-stage chemical reactions of the compound for which the physical property is predicted;
   a step of inputting synthesis information about the first stage chemical reaction of the compound into the first stage trained model, thereby predicting a physical property value of a product synthesized by the first stage chemical reaction of the compound;
   a step of repeating, from a second stage chemical reaction to a last stage chemical reaction of the compound, a process of inputting synthesis information about the n-th stage chemical reaction of the compound and synthesis result information about a product synthesized by the n-1-th stage chemical reaction of the compound, into the n-th stage trained model, thereby predicting a physical property value of a product synthesized by the n-th stage chemical reaction of the compound; and
   a step of outputting a predicted physical property value of a product synthesized by a last stage chemical reaction of the compound.

### EFFECTS OF THE INVENTION

According to the present disclosure, it is possible to accurately predict the physical properties of compounds synthesized by multi-stage chemical reactions.

### BRIEF DESCRIPTION OF DRAWINGS

[FIG. 1] FIG. 1 is a diagram illustrating a configuration of an example of an information processing system according to one embodiment.
[FIG. 2] FIG. 2 is a diagram illustrating a hardware configuration of an example of a computer according to one embodiment.
[FIG. 3] FIG. 3 is a diagram illustrating a functional configuration of an example of the information processing system according to one embodiment.
[FIG. 4A] FIG. 4A is an explanatory diagram illustrating an example of a process of generating a trained model according to one embodiment.
[FIG. 4B] FIG. 4B is an explanatory diagram illustrating an example of a process of generating a trained model according to one embodiment.
[FIG. 5A] FIG. 5A is an explanatory diagram illustrating an example of a process of predicting a physical property of a polymer using a trained model according to one embodiment.
[FIG. 5B] FIG. 5B is an explanatory diagram illustrating an example of a process of predicting a physical property of a polymer using a trained model according to one embodiment.
[FIG. 6] FIG. 6 is a flowchart illustrating an example of a processing procedure of a physical property prediction method performed by an information processing system according to one embodiment.
[FIG. 7] FIG. 7 is a diagram illustrating an image of an example of a synthesis information setting screen according to one embodiment.
[FIG. 8] FIG. 8 is a diagram illustrating an image of an example of a flow of data in a process of predicting a physical property of a polymer according to one embodiment.
[FIG. 9A] FIG. 9A is an explanatory diagram illustrating an example of a case where a structure of a finished product changes even when raw materials of a polymer and a composition ratio of the raw materials are the same.
[FIG. 9B] FIG. 9B is an explanatory diagram illustrating an example of a case where a structure of a finished product changes even when raw materials of a polymer and a composition ratio of the raw materials are the same.
[FIG. 10A]FIG. 10A is an explanatory diagram illustrating an example of a case where a structure of a finished product changes even when raw materials of a polymer and a composition ratio of the raw materials are the same.
[FIG. 10B]FIG. 10B is an explanatory diagram illustrating an example of a case where a structure of a finished product changes even when raw materials of a polymer and a composition ratio of the raw materials are the same.
[FIG. 11A]FIG. 11A is an explanatory diagram illustrating an example of a case where a structure of a finished product changes even when raw materials of a polymer and a composition ratio of the raw materials are the same.
[FIG. 11B]FIG. 11B is an explanatory diagram illustrating an example of a case where a structure of a finished product changes even when raw materials of a polymer and a composition ratio of the raw materials are the same.
[FIG. 12A]FIG. 12A is an explanatory diagram illustrating a process of generating a trained model according to one embodiment.
[FIG. 12B]FIG. 12B is an explanatory diagram illustrating a process of generating a trained model according to one embodiment.
[FIG. 13A]FIG. 13A is an explanatory diagram illustrating a process of predicting a physical property of a polymer using a trained model according to one embodiment.
[FIG. 13B]FIG. 13B is an explanatory diagram illustrating a process of predicting a physical property of a polymer using a trained model according to one embodiment.
[FIG. 14] FIG. 14 is a flowchart illustrating an example of a processing procedure of a physical property prediction method performed by an information processing system according to one embodiment.
[FIG. 15] FIG. 15 is a diagram illustrating an image of an example of a flow of data in a process of predicting a physical property of a polymer according to one embodiment.

### MODE OF CARRYING OUT THE INVENTION

Next, embodiments of the present invention will be described in detail. The present invention is not limited to the following embodiments. In the present embodiment, a polymer synthesized by multi-stage chemical reactions such as polymerization reactions and addition reactions (multi-stage synthesis) will be described, as an example of a compound synthesized by multi-stage chemical reactions. In the present embodiment, the multi-stage synthesis of the polymer includes: a pattern in which main/side chains are synthesized in multiple stages; a pattern in which raw materials (monomers and oligomers)/additives (a curing agent, a polymerization inhibitor, a catalyst, and a filler) are synthesized in multiple stages; and a pattern in which monomersxmonomers/another polymer/additives are synthesized in multiple stages.

### [First Embodiment]

### <System Configuration>

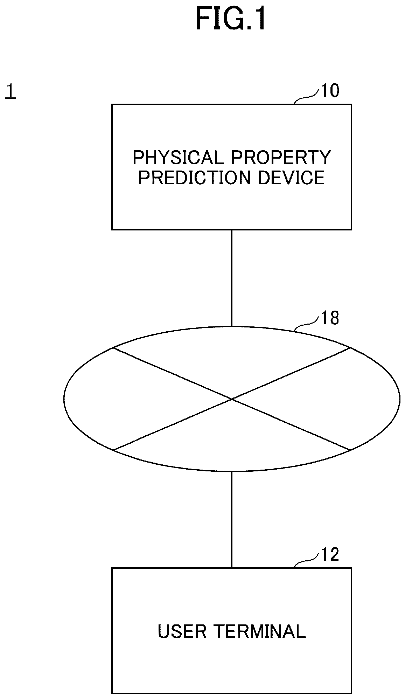

FIG. 1 is a diagram illustrating a configuration of an example of an information processing system according to one embodiment. An information processing system 1 of FIG. 1 includes a physical property prediction device 10 and a user terminal 12. The physical property prediction device 10 and the user terminal 12 are connected via a communication network 18, such as a local area network (LAN), the Internet, or the like, and are communicable with each other to perform data communication.

The user terminal 12 is an information processing terminal that can be operated by the user, such as a PC, a tablet terminal, a smartphone, or the like. The user terminal 12 receives input of information necessary for predicting physical properties of a polymer synthesized by multi-stage chemical reactions (hereinafter referred to simply as multi-stage synthesis) from the user, and causes the physical property prediction device 10 to predict the physical properties of the polymer. The user terminal 12 receives information such as the physical properties of the polymer predicted by the physical property prediction device 10, and presents the information to the user on a display device, for example.

The physical property prediction device 10 is an information processing device such as a PC for predicting the physical properties of the polymer. The physical property prediction device 10 generates a trained model by machine learning using training data. When the physical property prediction device 10 receives the information necessary for predicting the physical property of the polymer from the user terminal 12, the physical property of the polymer is predicted using the trained model. The physical property prediction device 10 transmits the predicted physical property of the polymer and other information to the user terminal 12.

The information processing system 1 illustrated in FIG. 1 is an example, and there are various examples of system configurations depending on the application and purpose. For example, the physical property prediction device 10 may be implemented by a plurality of computers or may be implemented as a cloud computing service. The information processing system 1 illustrated in FIG. 1 may be implemented by a stand-alone computer.

### <Hardware Configuration>

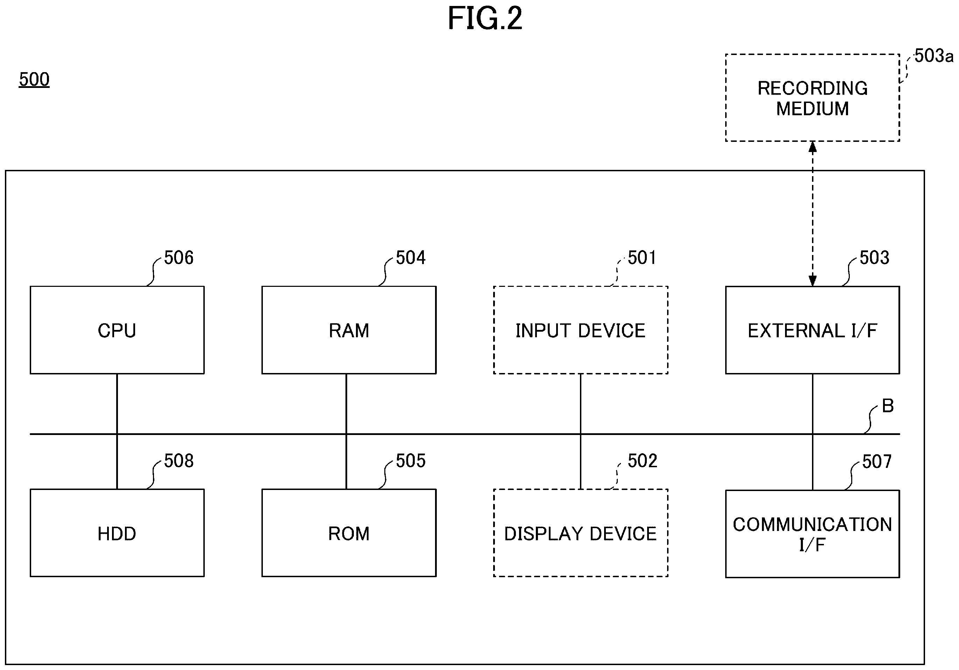

The physical property prediction device 10 and the user terminal 12 illustrated in FIG. 1 are implemented in a computer 500 having a hardware configuration illustrated in FIG. 2, for example.

FIG. 2 is a diagram illustrating the hardware configuration of an example of the computer according to the one embodiment. The computer 500 of FIG. 2 includes an input device 501, a display device 502, an external I/F 503, a RAM 504, a ROM 505, a CPU 506, a communication I/F 507, a HDD 508, or the like, which are connected to each other via a bus B. The input device 501 and the display device 502 may be connected and used when required.

The input device 501 is a touchscreen panel, operation keys or buttons, a keyboard, a mouse, or the like used by the user to input various signals. The display device 502 includes a display, such as a liquid crystal display, an organic EL display, or the like for displaying a screen, a speaker for outputting sound data, such as voice, sound, or the like, and the like. The communication I/F 507 is an interface used by the computer 500 to perform the data communication.

In addition, the HDD 508 is an example of a non-volatile storage device that stores programs and data. The stored programs and data include an operating system (OS) that is basic software for controlling the entire computer 500, applications for providing various functions on the OS, or the like. The computer 500 may use a drive device (for example, a solid state drive (SSD) or the like) using a flash memory as a storage media, in place of the HDD 508.

The external I/F 503 is an interface provided with respect to an external device. Examples of the external device include a recording medium 503a or the like. Thus, the computer 500 can read from and/or write to the recording medium 503a via the external I/F 503. Examples of the recording medium 503a include a flexible disk, a CD, a DVD, an SD memory card, a USB memory, or the like.

The ROM 505 is an example of a non-volatile semiconductor memory (storage device) that can retain programs and data even when the power is turned off. The ROM 505 stores programs and data, such as a BIOS that is executed when the computer 500 is started, OS settings, network settings, or the like. The RAM 504 is an example of a volatile semiconductor memory (storage device) that temporarily stores the programs and data.

The CPU 506 is an arithmetic unit that reads the programs and data from a storage device, such as the ROM 505, the HDD 508, or the like into the RAM 504, and executes a process to control the entire computer 500 or implement functions thereof. The computer 500 according to the one embodiment implements various functions of the physical property prediction device 10 and of the user terminal 12, which will be described later, by executing programs.

### <Functional Configuration>

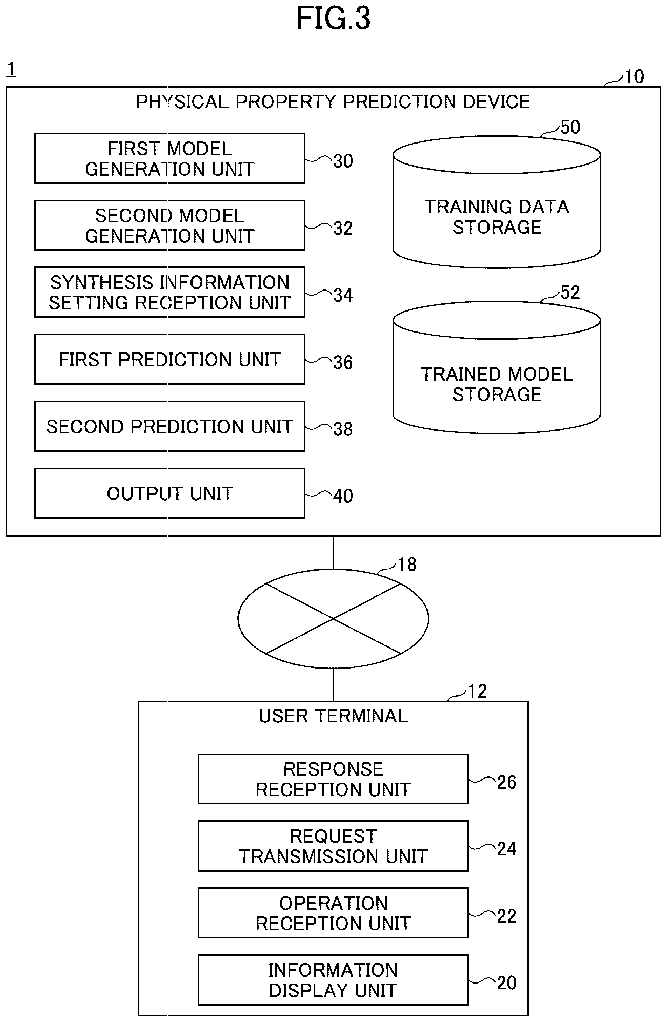

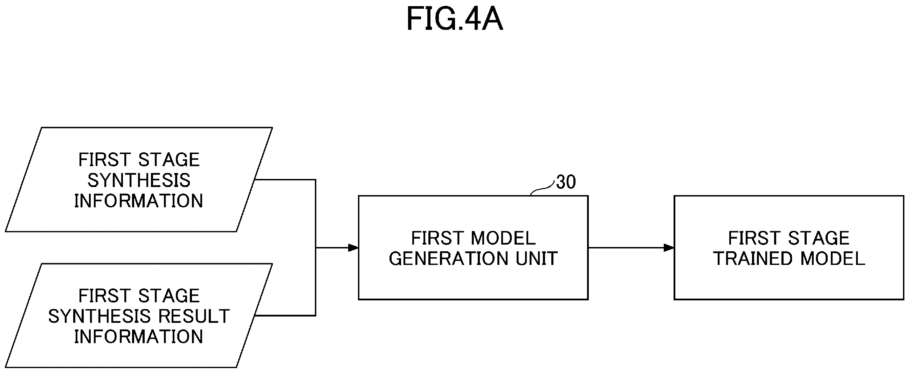

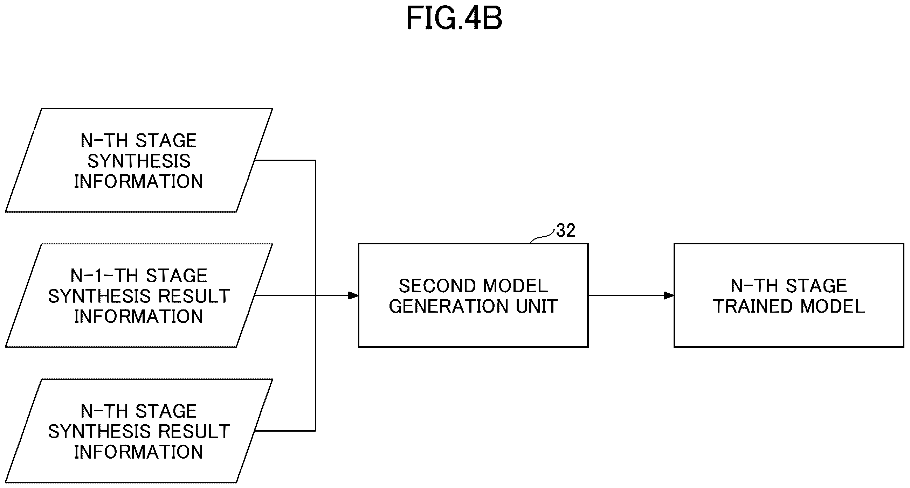

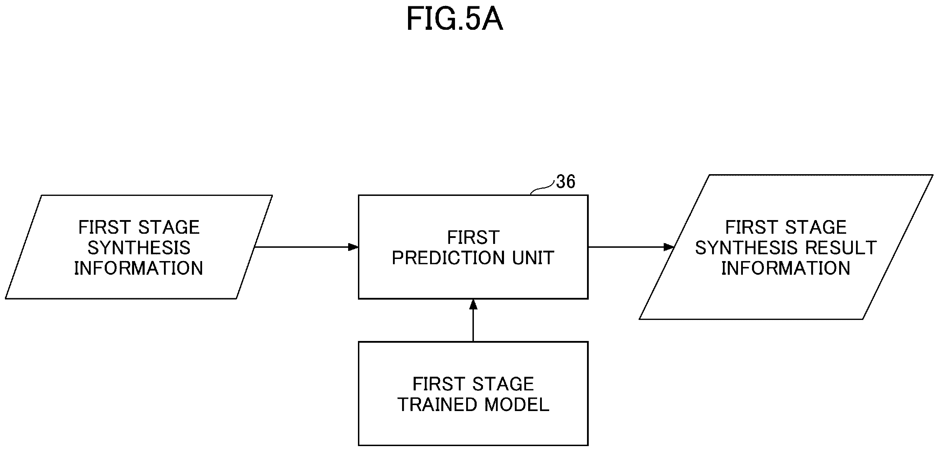

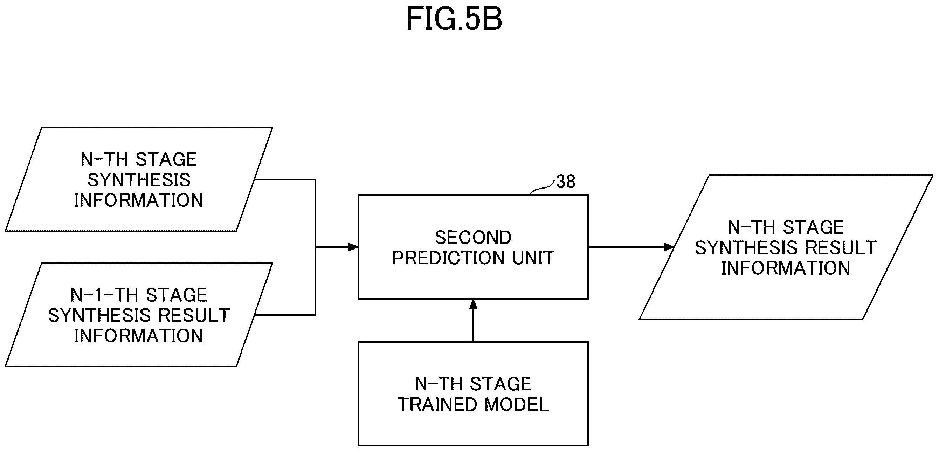

The configuration of the information processing system 1 according to the present embodiment will be described below. FIG. 3 is a diagram illustrating a functional configuration of an example of the information processing system according to the present embodiment. In the configuration diagram of FIG. 3, parts unnecessary for explaining the present embodiment are omitted as appropriate. FIGS. 4A and 4B are explanatory diagrams illustrating an example of a process of generating a trained model according to the present embodiment. FIGS. 5A and 5B are explanatory diagrams illustrating an example of a process of predicting a physical property of a polymer using the trained model according to the present embodiment.

The physical property prediction device 10 of the information processing system 1 illustrated in FIG. 3 includes a first model generation unit 30, a second model generation unit 32, a synthesis information setting reception unit 34, a first prediction unit 36, a second prediction unit 38, an output unit 40, a training data storage 50, and a trained model storage 52. The user terminal 12 includes an information display unit 20, an operation reception unit 22, a request transmission unit 24, and a response reception unit 26.

The information display unit 20 displays information presented to the user on the display device 502. The operation reception unit 22 receives operations from the user, such as input of information necessary for predicting the physical properties of the polymer to be synthesized in multiple stages. The request transmission unit 24 transmits a request for processing such as predicting the physical properties of the polymer, to the physical property prediction device 10. The response reception unit 26 receives a response to the request for processing such as predicting the physical properties of the polymer transmitted by the request transmission unit 24.

The first model generation unit 30 generates a first stage trained model, as illustrated in FIG. 4A, for example, by using training data in which synthesis information about the first stage chemical reaction among multi-stage chemical reactions and synthesis result information about a polymer, which is a product synthesized by the first stage chemical reaction, are associated with each other. The first stage trained model is a supervised trained model in which a correspondence between the synthesis information about the first stage chemical reaction and the synthesis result information about the polymer, which is a product synthesized by the first stage chemical reaction, is learned. As the supervised trained model, a supervised trained model such as a neural network or a genetic algorithm may be applied.

The synthesis information about the chemical reaction includes information on the composition ratio of raw materials such as the composition ratio of monomers and the composition ratio of a compound (an initiator, an inhibitor, and the like), and conditions such as synthesis temperature and synthesis rate. The synthesis result information includes a feature calculated from, as described below, the physical property value of the polymer synthesized by the chemical reaction and from the synthesis information.

The first model generation unit 30 generates the first stage trained model using training data stored in the training data storage 50. The first model generation unit 30 stores the generated first stage trained model in the trained model storage 52.

The second model generation unit 32 generates second stage through last stage trained models, for example, as illustrated in FIG. 4B, by using training data in which synthesis information about the n-th stage (n≥2) chemical reaction among multi-stage chemical reactions, synthesis result information about a polymer, which is a product synthesized by the n-th stage chemical reaction, and synthesis result information about a polymer, which is a product synthesized by the n-1-th stage chemical reaction, are associated with each other. The n-th stage trained model is a supervised trained model in which a correspondence between the synthesis information about the n-th stage chemical reaction, the synthesis result information about the polymer, which is a product synthesized by the n-th stage chemical reaction, and the synthesis result information about the polymer, which is a product synthesized by the n-1-th stage chemical reaction, is learned.

For example, the synthesis result information about the polymer synthesized by the n-1-th stage chemical reaction is a physical property value and a feature of the polymer synthesized by the n-1-th stage chemical reaction. The synthesis result information about the polymer synthesized by the n-1-th stage chemical reaction may include, in addition to the physical property value and the feature of the polymer synthesized by the n-1-th stage chemical reaction, the synthesis information, and may be other combinations of the physical property value, the feature, and the synthesis information.

The second model generation unit 32 generates the second stage through last stage trained models by using the training data stored in the training data storage 50. The second model generation unit 32 stores the second stage trained model to the last stage trained model that are generated, in the trained model storage 52. As the synthesis result information about the polymer, which is a product synthesized by a chemical reaction after the second stage, the synthesis result information predicted using the n-1-th stage trained model before the n-th stage, which is the stage to be predicted, may be used.

The synthesis information setting reception unit 34 receives a synthesis information setting for each of multi-stage chemical reactions of the polymer for which a physical property is to be predicted by the user. The synthesis information setting reception unit 34 may receive a composition ratio of raw materials such as monomers and compounds as a setting of a composition ratio range, and may automatically generate composition ratio information from a combination of composition ratios falling within the composition ratio range.

As illustrated in FIG. 5A, for example, the first prediction unit 36 inputs synthesis information about the first stage chemical reaction into the first stage trained model, and thereby predicts a physical property value of the polymer synthesized by the first stage chemical reaction. The first prediction unit 36 calculates a feature from the synthesis information about the predicted polymer. The first prediction unit 36 outputs the predicted physical property value and the calculated feature as first stage synthesis result information. Thus, the first stage synthesis result information includes the predicted physical property value of the polymer as well as the feature that can be calculated from the synthesis information.

As illustrated in FIG. 5B, for example, the second prediction unit 38 inputs synthesis information about the n-th stage chemical reaction and synthesis result information about the polymer synthesized by the n-1-th stage chemical reaction, into the n-th stage trained model, and thereby predicts a physical property value of the polymer synthesized by the n-th stage chemical reaction. The second prediction unit 38 calculates a feature from the synthesis information about the predicted polymer. The second prediction unit 38 outputs the predicted physical property value and the calculated feature as n-th stage synthesis result information. Thus, the n-th stage synthesis result information includes the predicted physical property value of the polymer as well as the feature that can be calculated from the synthesis information. The second prediction unit 38 repeats, from the second stage chemical reaction to the last stage chemical reaction, the process of predicting the n-th stage synthesis result information.

For example, the second prediction unit 38 inputs the synthesis information about the second stage chemical reaction and the first stage synthesis result information output from the first prediction unit 36 into the second stage trained model and outputs the predicted physical property value of the polymer synthesized by the second stage chemical reaction. The second prediction unit 38 calculates a feature from the synthesis information about the predicted polymer. The second prediction unit 38 outputs the predicted physical property value and the calculated feature as second stage synthesis result information.

The second prediction unit 38 inputs synthesis information about the third stage chemical reaction and the second stage synthesis result information into the third stage trained model, and thereby predicts the physical property value of the polymer synthesized by the third stage chemical reaction. The second prediction unit 38 calculates a feature from the synthesis information about the predicted polymer. The second prediction unit 38 outputs the predicted physical property value and the calculated feature as third stage synthesis result information. The second prediction unit 38 repeats such processing until the last stage chemical reaction.

Then, the output unit 40 transmits the physical property value of the polymer synthesized by the last stage chemical reaction predicted by the second prediction unit 38, in response to the request for predicting the physical property of the polymer from the user terminal 12. The configuration diagram of FIG. 3 is an example. The configuration of the information processing system 1 according to the present embodiment may be considered in various ways.

### <Feature Calculated from Synthesis information about Polymer>

In the present embodiment, examples of the feature that can be calculated from the synthesis information about the polymer includes a model structure, a number of substructures, a number of atoms in the model structure, and a number density of the substructures of the polymer, which is an example of a product.

The model structure is generated by repeating the process of duplicating and connecting structural units (repeat units) that constitute the polymer, by repetition a predetermined number of times. The polymer is represented by connecting multiple repeat units.

The number of substructures is calculated based on the model structure and is represented by an n-dimensional vector (a vector of the number of substructures of the model structure). The vector of the number of substructures is represented in n-dimensions by structural descriptors such as Extended Circular FingerPrint (ECFP), Atom Pair, Topological Torsion, Neural FingerPrint, and the like.

For example, when the model structure is "CCOCCOCCOCCOCCOCCOCCOCCOCCOCCO", the number of substructures is represented in n-dimensions by structural descriptors such as the number of C atoms, the number of 0 atoms, the number of COCs, and the like.

The number of atoms in the model structure is the number of atoms included in the model structure. When the model structure is "CCOCCOCCOCCOCCOCCOCCOCCOCCOCCO", the number of atoms included in the model structure is "30".

The number density of the substructures is calculated by the following equation (1) using the number of substructures and the number of atoms in the model structure. The calculated number density of the substructures is represented by the n-dimensional vector, the same as the number of substructures.

number density of substructures = number of substructures a number of atoms in model structure

The number density of the substructures may be obtained as follows. For example, when the model structure is "CCOCCOCCOCCOCCOCCOCCOCCOCCOCCO", the density of C atoms is "2/3(=20/30)", the density of O atoms is "1/3(=10/30)", ..., the density of COC is "9/30", and the like, by dividing each value of the vector of the number of substructures by the number of atoms in the model structure "30".

The details of the model structure, the number of substructures, the number of atoms in the model structure, and the number density of the substructures of the polymer are described, for example, in Japanese Patent No. 6633820. Thus, the feature used in the present embodiment can be calculated from the model structure of monomers and polymers included in the synthesis information about chemical reactions.

### <Physical Property Value of Polymer Predicted by Trained model>

In the present embodiment, the physical property value of the polymer predicted by the trained model includes viscosity, glass transition point, color difference, molecular weight, acid value, developability (speed, development form, and development residue), adhesiveness, transparency, sensitivity, solvent resistance (for example, NMP resistance), dispersibility, heat resistance (heat content phenomenon), heat resistance (yellowing color difference), and the like.

### <Processing>

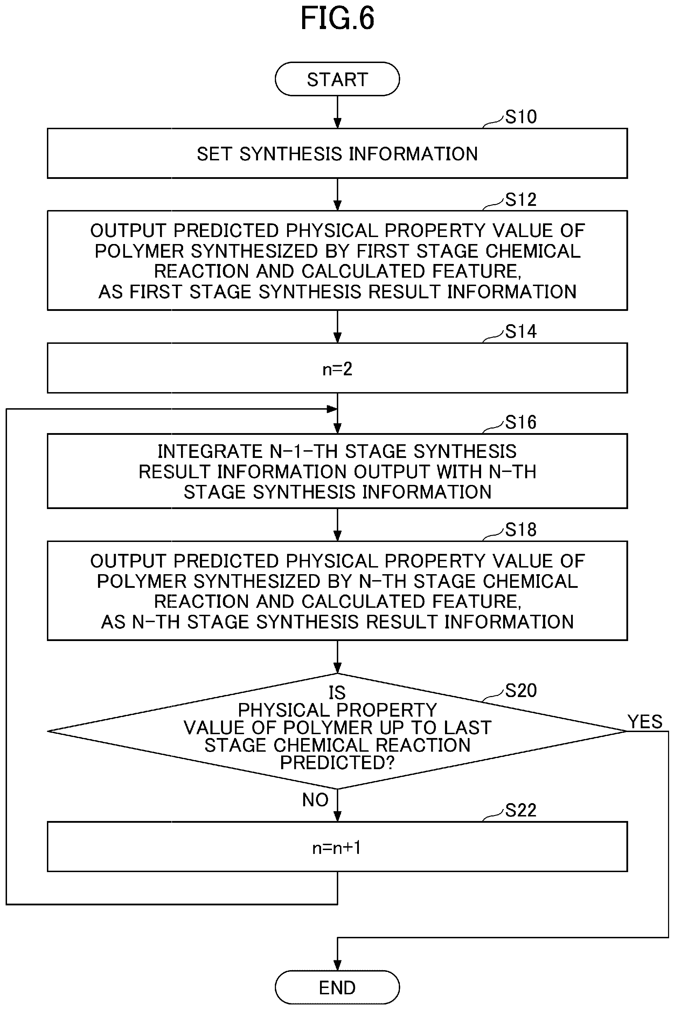

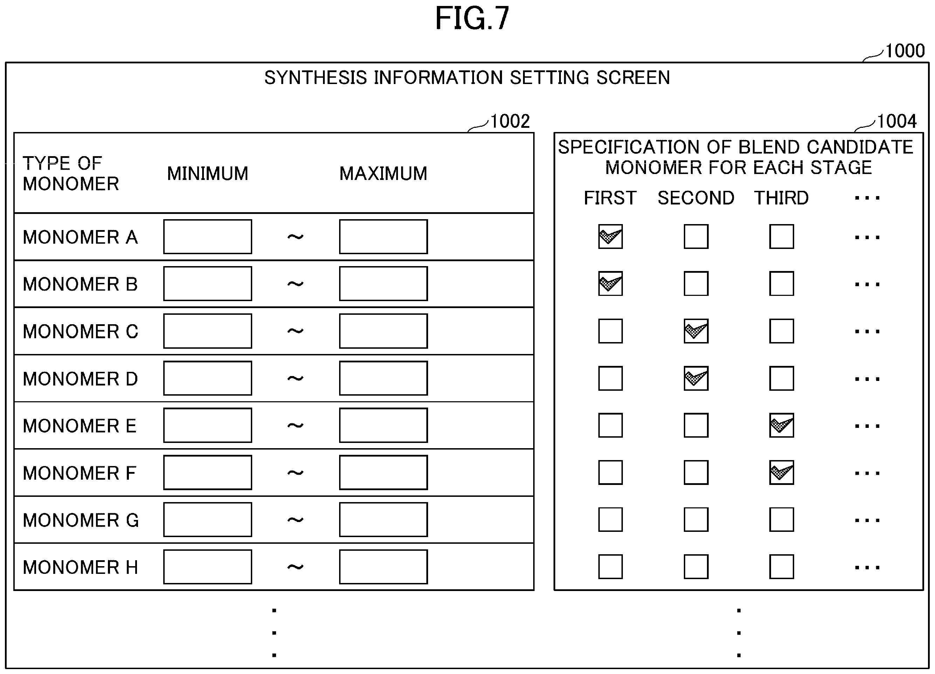

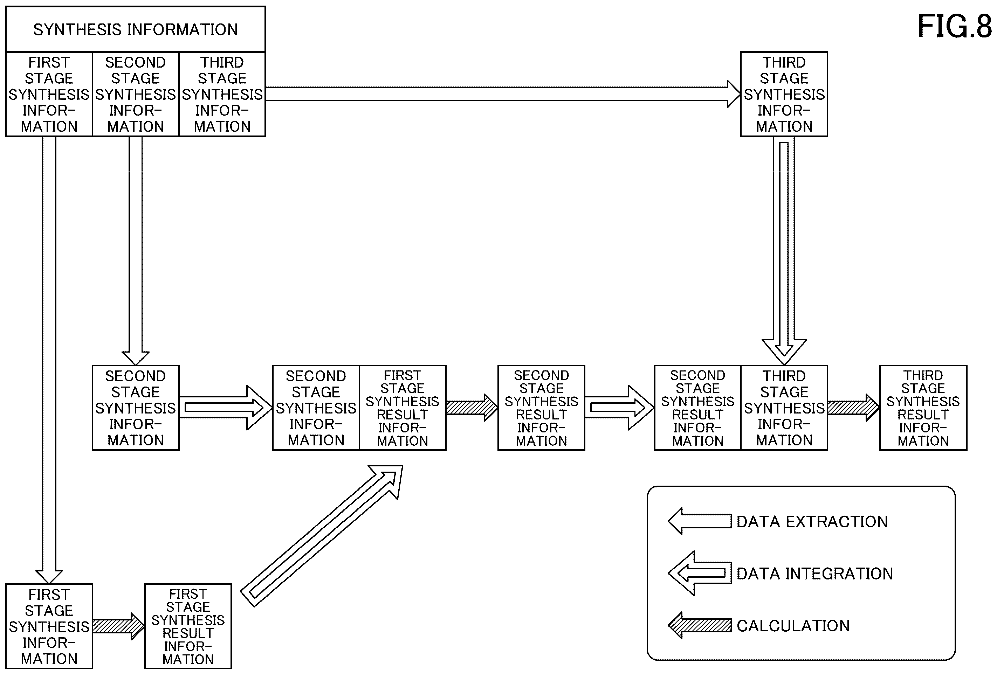

FIG. 6 is a flowchart illustrating an example of a processing procedure of a physical property prediction method performed by the information processing system according to the present embodiment. The process illustrated in the flowchart of FIG. 6 represents a physical property prediction process for predicting a physical property of a polymer to be synthesized in multiple stages, after generating a trained model. FIG. 7 is a diagram illustrating an image of an example of a synthesis information setting screen according to the present embodiment. FIG. 8 is a diagram illustrating an image of an example of a flow of data in a process of predicting a physical property of a polymer according to the present embodiment.

In step S10, the synthesis information setting reception unit 34 of the physical property prediction device 10 displays, for example, a synthesis information setting screen 1000 illustrated in FIG. 7 on the information display unit 20 of the user terminal 12, and receives a synthesis information setting for each of the multi-stage chemical reactions of the polymer for which the physical property is to be predicted by the user.

In the synthesis information setting screen 1000, a list of pre-prepared monomers that are raw materials (synthetic materials) for the polymer to be synthesized in multiple stages is displayed in a field 1002, and the minimum and maximum values of the composition ratio range can be entered for each monomer type. The synthesis information setting screen 1000 is an example of a screen for setting synthesis information for the polymer to be synthesized in multiple stages from a plurality of monomer types. A field 1004 is displayed in which monomers that are candidates for raw materials for each stage can be designated, as a composition candidate, from the monomer list displayed in the field 1002.

In the example illustrated in FIG. 7, monomer A and monomer B are designated as candidates for raw materials for the first stage, monomer C and monomer D are designated as candidates for raw materials for the second stage, and monomer E and monomer F are designated as candidates for raw materials for the third stage. For example, in the synthesis information setting screen 1000 illustrated in FIG. 7, a combination of a plurality of composition ratios is calculated at random or at a predetermined step size within the composition ratio ranges of monomer A and monomer B designated as candidates for raw materials for the first stage.

For example, in the synthesis information setting screen 1000 illustrated in FIG. 7, a combination of a plurality of composition ratios with the polymer (such as a main chain polymer) synthesized by the first stage chemical reaction is calculated at random or at a predetermined step size within the composition ratio ranges of monomer C and monomer D designated as candidates for raw materials for the second stage.

For example, in the synthesis information setting screen 1000 illustrated in FIG. 7, a combination of a plurality of composition ratios with the polymer (such as a graft polymer) synthesized by the second stage chemical reaction is calculated at random or at a predetermined step size within the composition ratio ranges of monomer E and monomer F designated as candidates for raw materials for the third stage.

The operation reception unit 22 of the user terminal 12 receives, from the user, input operations of synthesis information for each of the multi-stage chemical reactions as information necessary for predicting the physical property of the polymer to be synthesized in the multiple stages. The request transmission unit 24 requests the physical property prediction device 10 to predict the physical property of the polymer in accordance with the synthesis information for each of the multi-stage chemical reactions received by the operation reception unit 22.

The first prediction unit 36 of the physical property prediction device 10 requested to predict the physical property of the polymer from the user terminal 12 inputs the synthesis information about the first stage chemical reaction into the first stage trained model, as illustrated in FIG. 5A, for example, in step S12, and thereby predicts the physical property value of the polymer synthesized by the first stage chemical reaction. The first prediction unit 36 calculates a feature from the synthesis information about the predicted polymer. The first prediction unit 36 outputs the predicted physical property value and the calculated feature as the first stage synthesis result information.

For example, as illustrated in FIG. 8, the first prediction unit 36 extracts the first stage synthesis information from the synthesis information about each of the multi-stage chemical reactions, inputs the extracted first stage synthesis information into the first stage trained model, and thereby predicts the physical property value of the polymer synthesized by the first stage chemical reaction. The first prediction unit 36 calculates a feature of the polymer synthesized by the first stage chemical reaction, from the extracted first stage synthesis information. The first prediction unit 36 outputs the predicted physical property value and the calculated feature as the first stage synthesis result information.

In step S14, the second prediction unit 38 assigns "2" to "n". Proceeding to step S16, for example, the second prediction unit 38, as illustrated in FIG. 8, extracts the second stage synthesis information from the synthesis information about each of the multi-stage chemical reactions. The second prediction unit 38 integrates the first stage synthesis result information output by the first prediction unit 36 with the extracted second stage synthesis information.

In step S18, as illustrated in FIG. 5B, for example, the second prediction unit 38 inputs the synthesis information about the second stage chemical reaction and the first stage synthesis result information into the second stage trained model, and thereby predicts the physical property value of the polymer synthesized by the second stage chemical reaction. The second prediction unit 38 calculates a feature from the synthesis information about the predicted polymer. The second prediction unit 38 outputs the predicted physical property value and the calculated feature as the second stage synthesis result information.

For example, as illustrated in FIG. 8, the second prediction unit 38 inputs the integrated first stage synthesis result information and the second stage synthesis information into the second stage trained model to predict the physical property value of the polymer synthesized by the second stage chemical reaction. The second prediction unit 38 calculates a feature of the polymer synthesized by the second stage chemical reaction, from the second stage synthesis information. The second prediction unit 38 outputs the predicted physical property value and the calculated feature as the second stage synthesis result information.

In step S20, the second prediction unit 38 determines whether the physical property values of the polymer have been predicted up to the last stage chemical reaction. When the physical property values of the polymer have not been predicted up to the last stage chemical reaction, the second prediction unit 38 proceeds to step S22, adds "1" to "n", and repeats the processing of steps S16 to S20.

Here, an example in which the third stage chemical reaction is the last stage chemical reaction will be explained. Returning to step S16, for example, the second prediction unit 38 extracts the third stage synthesis information from the synthesis information for each of the multi-stage chemical reactions as illustrated in FIG. 8. Then, the second prediction unit 38 integrates the second stage synthesis result information output by the second prediction unit 38 with the extracted third stage synthesis information.

In step S18, as illustrated in FIG. 5B, for example, the second prediction unit 38 inputs the synthesis information about the third stage chemical reaction and the second stage synthesis result information into the third stage trained model, and thereby predicts the physical property value of the polymer synthesized by the third stage chemical reaction. The second prediction unit 38 calculates a feature from the synthesis information about the predicted polymer. The second prediction unit 38 outputs the predicted physical property value and the calculated feature as the third stage synthesis result information.

For example, as illustrated in FIG. 8, the second prediction unit 38 inputs the integrated synthesis result information from the second stage to the third stage into the third stage trained model, and thereby predicts the physical property value of the polymer synthesized by the third stage chemical reaction. The second prediction unit 38 calculates a feature of the polymer synthesized by the third stage chemical reaction, from the third stage synthesis information. The second prediction unit 38 outputs the predicted physical property value and the calculated feature as the third stage synthesis result information.

In step S20, the second prediction unit 38 determines whether the physical property values of the polymer have been predicted up to the last stage chemical reaction. When the physical property values of the polymer have been predicted up to the last stage chemical reaction, the second prediction unit 38 terminates the processing of the flowchart in FIG. 6.

### <Examples of Changes in Structure of Polymer to be Synthesized in Multiple Stages>

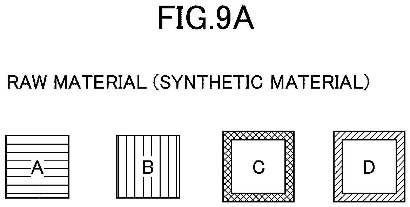

The physical property of the polymer synthesized in multiple stages by chemical reactions such as polymerization reactions and addition reactions may differ depending on the order of the chemical reactions, as illustrated in FIGS. 9A, 9B, 10A, 10B, 11A, and 11B as an example, even when the raw materials of the polymer and the composition ratio of the raw materials are the same.

FIGS. 9A, 9B, 10A, 10B, 11A, and 11B are explanatory diagrams of an example of a case where the structure of a finished product (a final product) changes even when the raw materials of the polymer and the composition ratio of the raw materials are the same. FIGS. 9A, 9B, 10A, 10B, 11A, and 11B are examples where the raw materials (synthetic materials) of the polymer are raw materials A, B, C, and D, as illustrated in FIG. 9A.

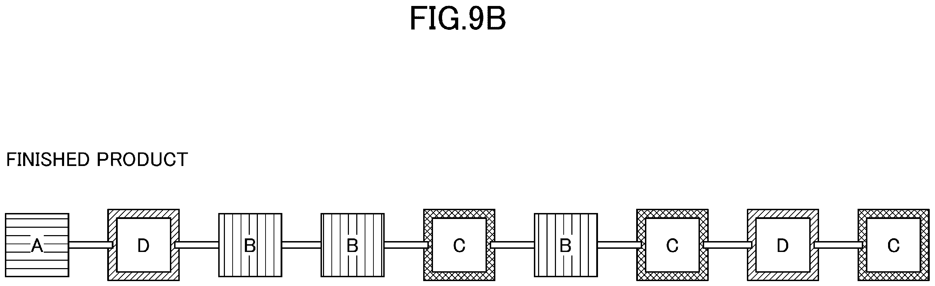

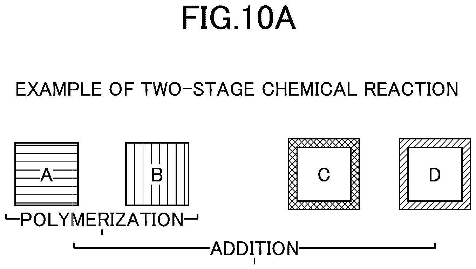

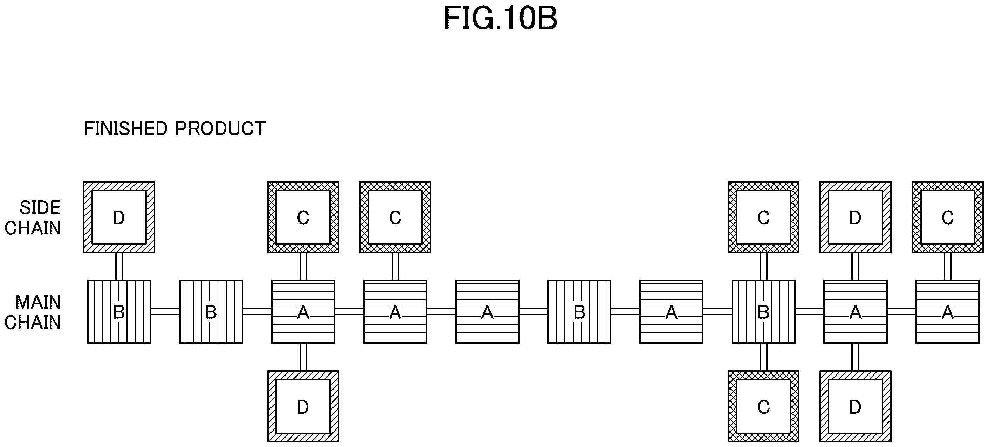

Even when the synthesis is completed by a one-stage chemical reaction and the composition ratio of raw materials A to D are the same, the composition ratio of the finished product, the polymer, may be different from the composition ratio of the raw material, resulting in, for example, the structure illustrated in FIG. 9B. FIG. 10A illustrates an example of synthesis by a two-stage chemical reaction. The finished product, the polymer, illustrated in FIG. 10A, synthesized by a two-stage chemical reaction, has the structure illustrated in FIG. 10B, for example. As illustrated in FIG. 10B, the finished product, the polymer, polymerizes the main chain of raw materials A and B in the first stage chemical reaction, and raw materials C and D are added as side chains to the main chain in the second stage chemical reaction.

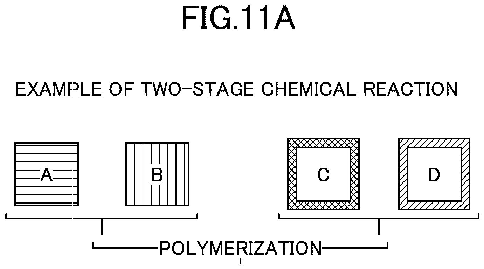

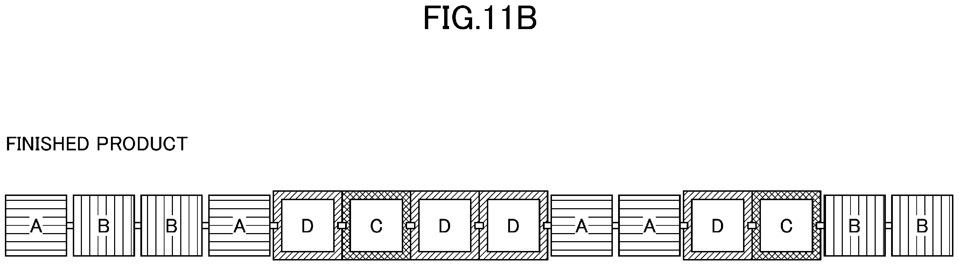

FIG. 11A illustrates an example of synthesis by a two-stage chemical reaction. The finished product, the polymer, illustrated in FIG. 11A, synthesized by a two-stage chemical reaction, has the structure illustrated in FIG. 11B, for example. For example, the finished product, the polymer, illustrated in FIG. 11A, synthesized by the two-stage chemical reaction, has a high probability that raw materials A and B are adjacent to each other and that raw materials C and D are adjacent to each other. As illustrated in FIGS. 9A, 9B, 10A, 10B, 11A, and 11B, because the structure of the finished product changes in the polymer synthesized in multiple stages, it is not possible to respond to changes in the structure of the finished product and to accurately predict the physical property of the finished product without using a trained model corresponding to each of the chemical reactions in multiple stages.

In addition, for polymers synthesized in multiple stages, the physical property of a product in the process (an intermediate), which differs from the target physical property of the finished product, may be an important factor in the target physical property of the finished product. By using a model that predicts the physical property different from the target physical property of the finished product with the intermediate, important factors of the target physical property of the finished product are not eliminated, and the physical property of the polymer synthesized in multiple stages can be accurately predicted.

Therefore, in the present embodiment, when the physical property of the polymer to be synthesized in multiple stages is predicted, a trained model for each of the multi-stage chemical reactions is generated and the physical property of the polymer is predicted. Accordingly, the physical property of the finished product can be accurately predicted. The physical property predicted by the trained model for each of the multi-stage chemical reactions is not limited to the target physical property of the finished product. For example, the trained model for predicting the physical property of the intermediate may include the trained model for predicting physical properties other than the target physical property of the finished product.

### [Second Embodiment]

In the second embodiment, the type of information used for generating the trained model and predicting the physical property of the product using the trained model is increased compared with the first embodiment, and the physical property of the finished product is predicted more accurately. Because the second embodiment is similar to the first embodiment except for some parts, description of the same parts will be omitted as appropriate.

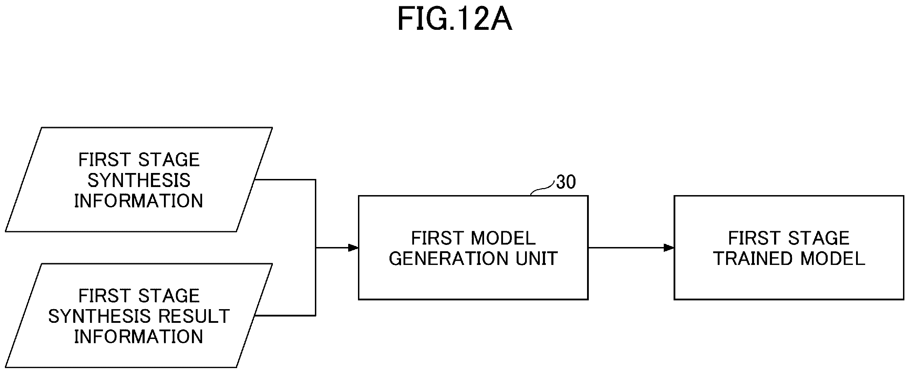

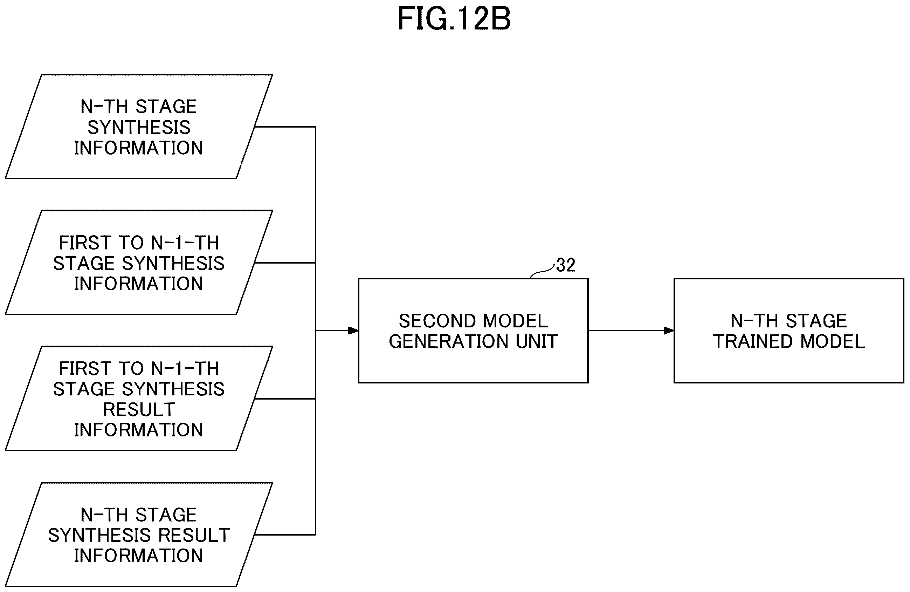

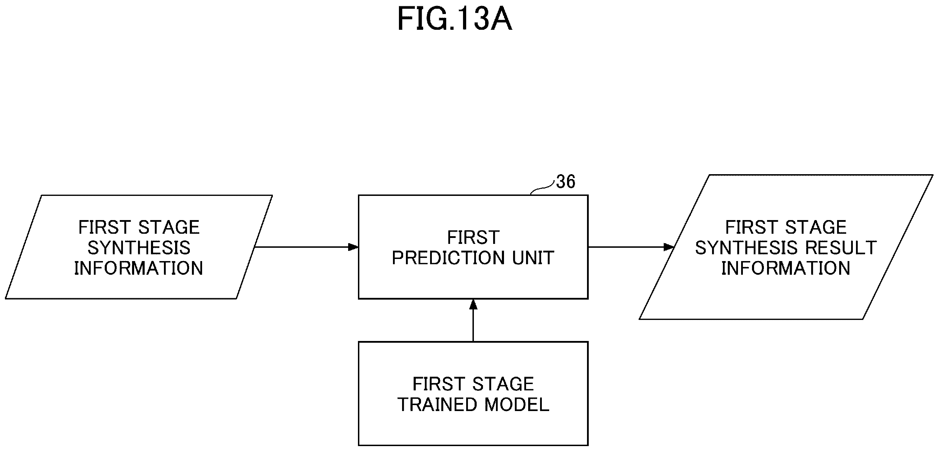

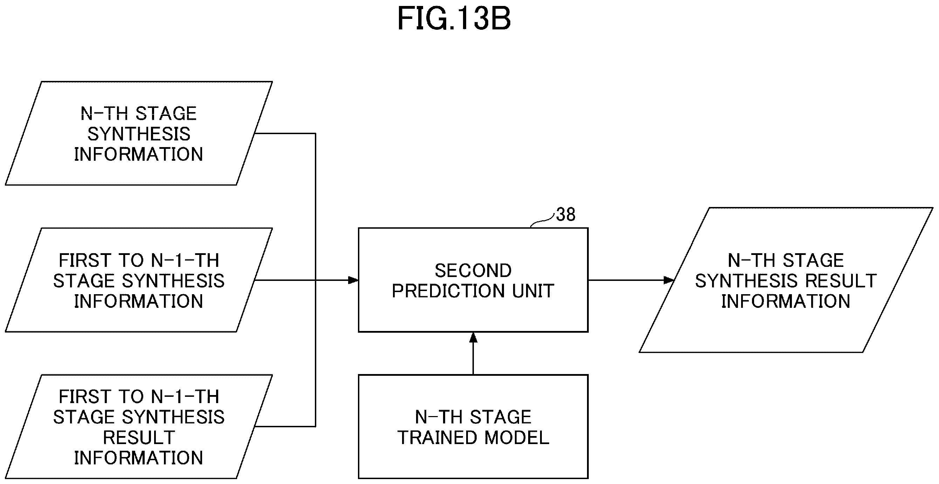

The system configuration and the hardware configuration are the same as those of the first embodiment. Because some of the functional configurations are different from those of the first embodiment, differences from those of the first embodiment will be described. FIGS. 12A and 12B are explanatory diagrams of an example of a process of generating a trained model according to the present embodiment. FIGS. 13A and 13B are explanatory diagrams of an example of a process of predicting the physical property of a polymer using the trained model according to the present embodiment.

The first model generation unit 30 generates a first stage trained model, as illustrated in FIG. 12A, for example, by using training data in which synthesis information about the first stage chemical reaction among multi-stage chemical reactions and synthesis result information about a polymer, which is a product synthesized by the first stage chemical reaction, are associated with each other. The first stage trained model is a supervised trained model in which a correspondence between the synthesis information about the first stage chemical reaction and the synthesis result information about the polymer, which is a product synthesized by the first stage chemical reaction, is learned.

The first model generation unit 30 generates the first stage trained model using training data stored in the training data storage 50. The first model generation unit 30 stores the generated first stage trained model in the trained model storage 52.

The second model generation unit 32 generates second stage through last stage trained models, for example, as illustrated in FIG. 12B, by using training data in which synthesis information about the n-th stage chemical reaction, synthesis result information about a polymer, which is a product synthesized by the n-th stage chemical reaction, synthesis information about first stage through n-1-th stage chemical reactions, and synthesis result information about a polymer, which is a product synthesized by the first stage through n-1-th stage chemical reactions, are associated with each other. The n-th stage trained model is a supervised trained model in which a correspondence between the synthesis information about the n-th stage chemical reaction, the synthesis result information about the polymer, which is a product synthesized by the n-th stage chemical reaction, the synthesis information about the chemical reactions from the first stage chemical reaction to the n-1-th stage chemical reaction, and the synthesis result information about the polymer, which is a product synthesized by the chemical reactions from the first stage chemical reaction to the n-1-th stage chemical reaction, is learned.

For example, the synthesis result information about the polymer synthesized by the chemical reactions from the first stage chemical reaction to the n-1-th stage chemical reaction is the physical property and the feature of the polymer synthesized by each of the chemical reactions from the first stage chemical reaction to the n-1-th stage chemical reaction.

The second model generation unit 32 generates the second stage through last stage trained models by using the training data stored in the training data storage 50. The second model generation unit 32 stores the second stage trained model to the last stage trained model that are generated, in the trained model storage 52.

As illustrated in FIG. 13A, for example, the first prediction unit 36 inputs synthesis information about the first stage chemical reaction into the first stage trained model, and thereby predicts the physical property value of the polymer synthesized by the first stage chemical reaction. The first prediction unit 36 calculates a feature from the synthesis information about the predicted polymer. The first prediction unit 36 outputs the predicted physical property value and the calculated feature as first stage synthesis result information. Thus, the first stage synthesis result information includes the predicted physical property value of the polymer as well as the feature that can be calculated from the synthesis information.

As illustrated in FIG. 13B, for example, the second prediction unit 38 inputs synthesis information about the n-th stage chemical reaction, synthesis information about the first stage chemical reaction to the n-1-th stage chemical reaction, and synthesis result information about the polymer synthesized by the first stage chemical reaction to the n-1-th stage chemical reaction, into the n-th stage trained model, and thereby predicts a physical property value of the polymer synthesized by the n-th stage chemical reaction. The second prediction unit 38 calculates a feature from the synthesis information about the predicted polymer. The second prediction unit 38 outputs the predicted physical property value and the calculated feature as n-th stage synthesis result information. Thus, the n-th stage synthesis result information includes the predicted physical property value of the polymer as well as the feature that can be calculated from the synthesis information. The second prediction unit 38 repeats, from the second stage chemical reaction to the last stage chemical reaction, the process of predicting the n-th stage synthesis result information.

For example, the second prediction unit 38 inputs the synthesis information about the second stage chemical reaction, the synthesis information about the first stage chemical reaction, and the first stage synthesis result information output from the first prediction unit 36 into the second stage trained model, and thereby predicts the physical property value of the polymer synthesized by the second stage chemical reaction. The second prediction unit 38 calculates a feature from the synthesis information about the predicted polymer. The second prediction unit 38 outputs the predicted physical property value and the calculated feature as second stage synthesis result information.

The second prediction unit 38 inputs synthesis information about the third stage chemical reaction, synthesis information about the chemical reactions of the first stage chemical reaction to the second stage chemical reaction, and synthesis result information about the first stage chemical reaction to the second stage chemical reaction into the third stage trained model, and thereby predicts the physical property value of the polymer synthesized by the third stage chemical reaction. The second prediction unit 38 calculates a feature from the synthesis information about the predicted polymer. The second prediction unit 38 outputs the predicted physical property value and the calculated feature as third stage synthesis result information. The second prediction unit 38 repeats such processing until the last stage chemical reaction.

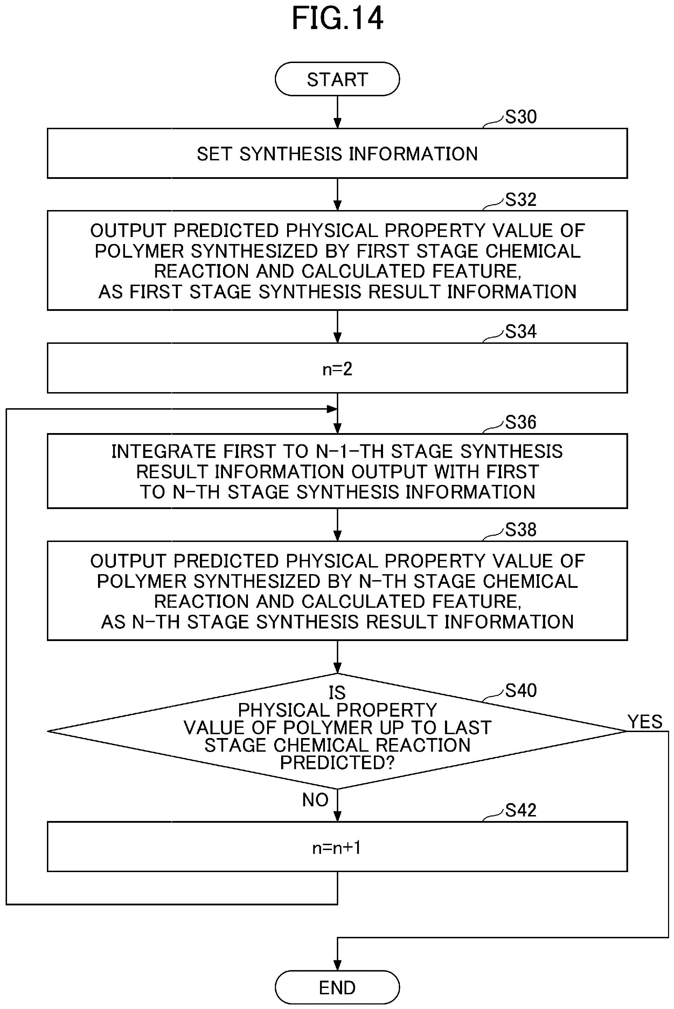

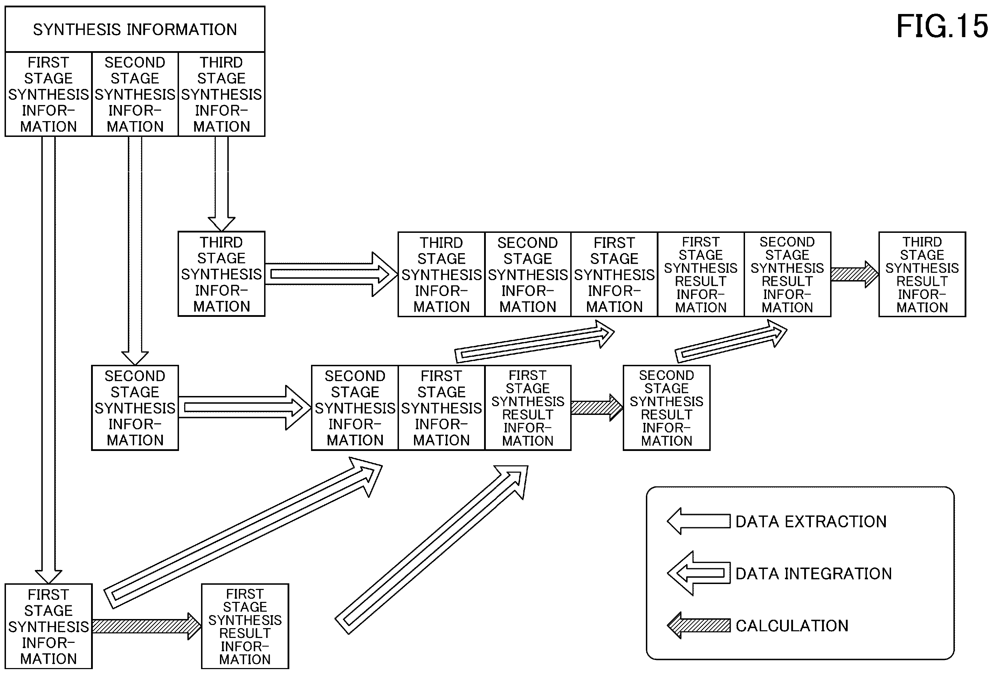

FIG. 14 is a flowchart illustrating an example of a processing procedure of a physical property prediction method performed by the information processing system according to the present embodiment. The process illustrated in the flowchart of FIG. 14, similar to the flowchart illustrated in FIG. 6, represents a physical property prediction process for predicting a physical property of a polymer to be synthesized in multiple stages, after generating a trained model. FIG. 15 is a diagram illustrating an image of an example of a flow of data in a process for predicting a physical property of a polymer according to the present embodiment.

In step S30, the synthesis information setting reception unit 34 of the physical property prediction device 10 displays, for example, the synthesis information setting screen 1000 illustrated in FIG. 7 on the information display unit 20 of the user terminal 12, and receives a synthesis information setting for each of the multi-stage chemical reactions of the polymer for which the physical property is to be predicted by the user.

The operation reception unit 22 of the user terminal 12 receives, from the user, input operations of synthesis information for each of the multi-stage chemical reactions as information necessary for predicting the physical property of the polymer to be synthesized in the multiple stages. The request transmission unit 24 requests the physical property prediction device 10 to predict the physical property of the polymer in accordance with the synthesis information for each of the multi-stage chemical reactions received by the operation reception unit 22.

The first prediction unit 36 of the physical property prediction device 10 requested to predict the physical property of the polymer from the user terminal 12 inputs the synthesis information about the first stage chemical reaction into the first stage trained model, as illustrated in FIG. 13A, for example, in step S32, and thereby predicts the physical property value of the polymer synthesized by the first stage chemical reaction. The first prediction unit 36 calculates a feature from the synthesis information about the predicted polymer. The first prediction unit 36 outputs the predicted physical property value and the calculated feature as the first stage synthesis result information.

For example, as illustrated in FIG. 15, the first prediction unit 36 extracts the first stage synthesis information from the synthesis information about each of the multi-stage chemical reactions, inputs the extracted first stage synthesis information into the first stage trained model, and thereby predicts the physical property value of the polymer synthesized by the first stage chemical reaction. The first prediction unit 36 calculates a feature of the polymer synthesized by the first stage chemical reaction, from the extracted first stage synthesis information. The first prediction unit 36 outputs the predicted physical property value and the calculated feature as the first stage synthesis result information.

In step S34, the second prediction unit 38 assigns "2" to "n". Proceeding to step S36, for example, the second prediction unit 38, as illustrated in FIG. 15, extracts the second stage synthesis information from the synthesis information about each of the multi-stage chemical reactions. The second prediction unit 38 integrates the first stage synthesis result information output by the first prediction unit 36 with the extracted synthesis information from the first stage to the second stage.

In step S38, as illustrated in FIG. 13B, for example, the second prediction unit 38 inputs the synthesis information about the second stage chemical reaction, the synthesis information about the first stage chemical reaction, and the first stage synthesis result information into the second stage trained model, and thereby predicts the physical property value of the polymer synthesized by the second stage chemical reaction. The second prediction unit 38 calculates a feature from the synthesis information about the predicted polymer. The second prediction unit 38 outputs the predicted physical property value and the calculated feature as the second stage synthesis result information.

For example, as illustrated in FIG. 15, the second prediction unit 38 inputs the integrated first stage synthesis result information and the synthesis information from the first stage to the second stage into the second stage trained model, and thereby predicts the physical property value of the polymer synthesized by the second stage chemical reaction. The second prediction unit 38 calculates a feature of the polymer synthesized by the second stage chemical reaction, from the second stage synthesis information. The second prediction unit 38 outputs the predicted physical property value and the calculated feature as the second stage synthesis result information.

In step S40, the second prediction unit 38 determines whether the physical property values of the polymer have been predicted up to the last stage chemical reaction. When the physical property values of the polymer have not been predicted up to the last stage chemical reaction, the second prediction unit 38 proceeds to step S42, adds "1" to "n", and repeats the processing of steps S36 to S40.

Here, an example in which the third stage chemical reaction is the last stage chemical reaction will be explained. Returning to step S36, for example, the second prediction unit 38 extracts the third stage synthesis information from the synthesis information for each of the multi-stage chemical reactions, as illustrated in FIG. 15. Then, the second prediction unit 38 integrates the first stage synthesis result information output by the first prediction unit 36, the second stage synthesis result information output by the second prediction unit 38, and the extracted synthesis information from the first stage to the third stage.

In step S38, as illustrated in FIG. 13B, for example, the second prediction unit 38 inputs the synthesis information about the chemical reactions from the first stage chemical reaction to the third stage chemical reaction and the synthesis result information about the chemical reactions from the first stage chemical reaction to the second stage synthesis result information, into the third stage trained model, and thereby predicts the physical property value of the polymer synthesized by the third stage chemical reaction. The second prediction unit 38 calculates a feature from the synthesis information about the predicted polymer. The second prediction unit 38 outputs the predicted physical property value and the calculated feature as the third stage synthesis result information.

For example, as illustrated in FIG. 15, the second prediction unit 38 inputs the integrated synthesis result information from the first stage to the second stage and the synthesis information from the first stage to the third stage into the third stage trained model, and thereby predicts the physical property value of the polymer synthesized by the third stage chemical reaction. The second prediction unit 38 calculates a feature of the polymer synthesized by the third stage chemical reaction, from the third stage synthesis information. The second prediction unit 38 outputs the predicted physical property value and the feature as the third stage synthesis result information.

In step S40, the second prediction unit 38 determines whether the physical property values of the polymer have been predicted up to the last stage chemical reaction. When the physical property values of the polymer have been predicted up to the last stage chemical reaction, the second prediction unit 38 terminates the processing of the flowchart illustrated in FIG. 14.

### [Other Embodiments]

The physical property of a compound such as a polymer predicted by the physical property prediction device 10 according to the present embodiment can be applied to a composition design device or a composition design support device for a compound to be synthesized in multiple stages, a program for implementing the composition design device or the composition design support device, and the like. The physical property prediction device 10 according to the present embodiment uses the physical property of a compound such as a predicted polymer. Therefore, synthesis information capable of producing a compound with a good target physical property can be selected from combinations of composition ratios that fall within the range of the raw material composition ratios set by the user. The physical property prediction device 10 according to the present embodiment may supply the selected synthesis information to a production apparatus that produces a compound such as a polymer based on input of synthesis information for a multi-stage synthesis, for example, to cause the production apparatus to produce a compound, such as a polymer.

The information processing system 1 according to the present embodiment can provide a physical property prediction device, a physical property prediction method, and a program capable of accurately predicting the physical properties of compounds synthesized by multi-stage chemical reactions.

Although the embodiments are described heretofore, various modifications to the form and details may be made without departing from the subject matter and scope of the appended claims.

Although the present invention is described with reference to the embodiments, the present invention is not limited to the embodiments described above, and various modifications can be made without departing from the scope of the subject matter recited in the claims. The present international application is based upon and claims priority to Japanese Patent Application No. 2021-104173, filed on June 23, 2021, the entire contents of which are incorporated herein by reference.

### DESCRIPTION OF REFERENCE NUMERALS

1 Information processing system
10 Physical property prediction device
12 User terminal
18 Communication network
20 Information display unit
22 Operation reception unit
24 Request transmission unit
26 Response reception unit
30 First model generation unit
32 Second model generation unit
34 Synthesis information setting reception unit
36 First prediction unit
38 Second prediction unit
40 Output unit
50 Training data storage
52 Trained model storage
502 Display device

## Claims

1. A physical property prediction device for predicting a physical property of a compound synthesized by multi-stage chemical reactions, the physical property prediction device comprising:
a first model generation unit configured to generate a first stage trained model for predicting a physical property value of a product synthesized by a first stage chemical reaction, by using, as training data, synthesis information about the first stage chemical reaction and synthesis result information about a product synthesized by the first stage chemical reaction;
a second model generation unit configured to generate second stage through last stage trained models for predicting a physical property value of a product synthesized by an n-th stage (n≥2) chemical reaction, by using, as training data, synthesis information about the n-th stage chemical reaction, synthesis result information about a product synthesized by the n-th stage chemical reaction, and synthesis result information about a product synthesized by an n-1-th stage chemical reaction;
a synthesis information setting reception unit configured to receive a synthesis information setting for each of the multi-stage chemical reactions of the compound for which the physical property is predicted;
a first prediction unit configured to input synthesis information about the first stage chemical reaction of the compound into the first stage trained model, thereby to predict a physical property value of a product synthesized by the first stage chemical reaction of the compound;
a second prediction unit configured to repeat, from a second stage chemical reaction to a last stage chemical reaction of the compound, a process of inputting synthesis information about the n-th stage chemical reaction of the compound and synthesis result information about a product synthesized by the n-1-th stage chemical reaction of the compound, into the n-th stage trained model, thereby predicting a physical property value of a product synthesized by the n-th stage chemical reaction of the compound; and
an output unit configured to output a predicted physical property value of a product synthesized by a last stage chemical reaction of the compound, predicted by the second prediction unit.

2. The physical property prediction device according to claim 1, wherein
the second model generation unit is configured to generate second stage through last stage trained models for predicting a physical property value of a product synthesized by each of the second stage through last stage chemical reactions, by using, as training data, synthesis information about the n-th stage chemical reaction, synthesis result information about a product synthesized by the n-th stage chemical reaction, synthesis information about a chemical reaction of a stage prior to the n-th stage, and synthesis result information about a product synthesized by the chemical reaction of the stage prior to the n-th stage, and
the second prediction unit is configured to repeat, to the last stage chemical reaction of the compound, a process of inputting synthesis information about chemical reactions from the first stage chemical reaction to the n-th stage chemical reaction of the compound and synthesis result information about each of products synthesized by chemical reactions from the first stage chemical reaction to the n-1-th stage chemical reaction of the compound, into the n-th stage trained model, thereby predicting the physical property value of the product synthesized by the n-th stage chemical reaction of the compound.

3. The physical property prediction device according to claim 1 or 2, wherein
the synthesis result information about the product includes a feature calculated from the predicted physical property value of the product synthesized by the chemical reaction and from the synthesis information about the product.

4. The physical property prediction device according to claim 3, wherein
the compound synthesized by the multi-stage chemical reactions is a polymer that is polymerized from multiple types of monomers, and
the feature includes at least one of a model structure, a number of substructures, a number of atoms in the model structure, and a number density of the substructures of the polymer.

5. The physical property prediction device according to claim 3 or 4, wherein
the physical property value includes, as information, at least one of viscosity, glass transition point, color difference, molecular weight, acid value, developability, adhesiveness, transparency, sensitivity, solvent resistance, dispersibility, and heat resistance of the product.

6. A physical property prediction method executed by a computer for predicting a physical property of a compound synthesized by multi-stage chemical reactions, the physical property prediction method comprising:
a step of generating a first stage trained model for predicting a physical property value of a product synthesized by a first stage chemical reaction, by using, as training data, synthesis information about the first stage chemical reaction and synthesis result information about a product synthesized by the first stage chemical reaction;
a step of generating second stage through last stage trained models for predicting a physical property value of a product synthesized by an n-th stage (n≥2) chemical reaction, by using, as training data, synthesis information about the n-th stage chemical reaction, synthesis result information about a product synthesized by the n-th stage chemical reaction, and synthesis result information about a product synthesized by an n-1-th stage chemical reaction;
a step of receiving a synthesis information setting for each of the multi-stage chemical reactions of the compound for which the physical property is predicted;
a step of inputting synthesis information about the first stage chemical reaction of the compound into the first stage trained model, thereby predicting a physical property value of a product synthesized by the first stage chemical reaction of the compound;
a step of repeating, from a second stage chemical reaction to a last stage chemical reaction of the compound, a process of inputting synthesis information about the n-th stage chemical reaction of the compound and synthesis result information about a product synthesized by the n-1-th stage chemical reaction of the compound, into the n-th stage trained model, thereby predicting a physical property value of a product synthesized by the n-th stage chemical reaction of the compound; and
a step of outputting a predicted physical property value of a product synthesized by a last stage chemical reaction of the compound.

7. A program for causing a computer for predicting a physical property of a compound synthesized by multi-stage chemical reactions, to execute:
a step of generating a first stage trained model for predicting a physical property value of a product synthesized by a first stage chemical reaction, by using, as training data, synthesis information about the first stage chemical reaction and synthesis result information about a product synthesized by the first stage chemical reaction;
a step of generating second stage through last stage trained models for predicting a physical property value of a product synthesized by an n-th stage (n≥2) chemical reaction, by using, as training data, synthesis information about the n-th stage chemical reaction, synthesis result information about a product synthesized by the n-th stage chemical reaction, and synthesis result information about a product synthesized by an n-1-th stage chemical reaction;
a step of receiving a synthesis information setting for each of the multi-stage chemical reactions of the compound for which the physical property is predicted;
a step of inputting synthesis information about the first stage chemical reaction of the compound into the first stage trained model, thereby predicting a physical property value of a product synthesized by the first stage chemical reaction of the compound;
a step of repeating, from a second stage chemical reaction to a last stage chemical reaction of the compound, a process of inputting synthesis information about the n-th stage chemical reaction of the compound and synthesis result information about a product synthesized by the n-1-th stage chemical reaction of the compound, into the n-th stage trained model, thereby predicting a physical property value of a product synthesized by the n-th stage chemical reaction of the compound; and
a step of outputting a predicted physical property value of a product synthesized by a last stage chemical reaction of the compound.
